# EUROPEAN PATENT APPLICATION

(11) **EP 4 260 855 A1**
(43) Date of publication of application: **18.10.2023**
(21) Application number: 22208963.3
(22) Date of filing: 22.11.2022
(51) Int. Cl.: A61K 31/353, A61P 25/28

(54) **GENISTEIN FOR USE IN THE PREVENTION OF MEMORY AND COGNITIVE LOSS**

(30) Priority: 11.04.2022 PL 44089622
(71) Applicant: Uniwersytet Gdanski, 80-309 Gdansk (PL)
(72) Inventor: Pierzynowska, Karolina, 80-281 Gdansk (PL); Cyske, Zuzanna, 80-281 Gdansk (PL); Gaffke, Lidia, 84-200 Wejherowo (PL); Podlacha, Magdalena, 80-363 Gdansk (PL); Wegrzyn, Grzegorz, 80-299 Gdansk (PL); Myslinska, Dorota, 83-000 Bedzieszyn (PL); Majkutewicz, Irena, 83-110 Tczew (PL)
(74) Representative: Pawlowska, Justyna

(57) **Abstract**

The invention involves genistein for use in preventing memory and cognitive loss in healthy people as a result of the gradual brain ageing processes that physiologically occur in healthy people with age, with genistein acting as an agent to prevent and reduce the deposition of harmful insoluble aggregates and protein deposits in the brain, especially beta-amyloid, and is administered orally.

## Description

The invention refers to the medical use of genistein to prevent the loss of memory and cognitive abilities of healthy people as a result of the gradual physiological ageing process of the brain as a person ages, without neurological diseases, by preventing and reducing the deposition of harmful and insoluble protein aggregates and deposits including beta amyloid in the brain in the process of physiological ageing,

Cognitive functions are defined as a set of mental processes - associated with the work of the brain, determining the correct reception, processing, encoding and use of stimuli, arriving from the external environment, by the central nervous system. Among the most important are: perception, attention and memory. It is well known that brain ageing processes negatively affect the efficiency of cognitive functions, resulting in a steadily increasing number of people in whom cognitive impairment of varying degrees or even dementia is confirmed. Between the gradual (physiological) loss of cognitive functions and dementia, there are many intermediate states characterised by a complexity of symptoms, which makes it difficult to make a quick and correct diagnosis. It is therefore important to publicise the use of appropriate preventive compounds early enough to prevent progressive loss of cognitive function. An important aspect of appropriate prevention is also to take into account the biological factors underlying the gender differences in memory disorders. As is well known, the female brain is on average 10 per cent smaller and a significant part of it is made up of grey matter, while in men the white matter and cerebrospinal fluid form the dominant part. Progressive ageing processes result in a reduction in the volume of brain tissue. Although these changes are slower in women than in men, the areas affected are key in terms of gradual loss of cognitive function. In men, greater loss of brain tissue is observed in the frontal and temporal regions, which translates into changes in behaviour. In women, on the other hand, the reduction in volume is significantly greater in the parietal regions and the hippocampus, resulting in a deterioration of memory processes, which are also significantly affected by the cycle and hormonal activity. Another important aspect is the body's exposure to stressful stimuli, which, especially in older individuals, results in impaired physiological functions, including learning and memory processes. Both chronic and incidental exposure to increased stressors leads to elevated concentrations of catecholamines, disruption of Ca²⁺ ion-dependent signalling pathwaysand protein kinase C, and degeneration of dendritic spines, which, combined with the reduced neuroplastic potential observed in older individuals, results in significant impairment of memory processes.

Effective methods are still being sought to prevent cognitive, memory and stress response declines that progress with age, without diagnosing central nervous system disease in a group of people who experience physiological brain ageing.

In the state of the art, one can find descriptions of the use of genistein in neurodegenerative diseases such as Alzhaimer's and Huntington's diseases, e.g. in EP3583942 where genistein is a stimulator of autophagy, in EP3300730 where it is effective in reducing levels of mutant huntingtin.

The potential use of genistein in the protection of the nervous system against oxidative stress in healthy individuals, one of the known processes causing damage to biological macromolecules and, as a result, accelerating neurodegeneration, is known, possibly as a phytoestrogen, mildly stimulating estrogen receptors, which may also have a protective role against external, harmful factors causing cell damage. Nevertheless, the state of the art described, in terms of the action of genistein in healthy individuals, reflects the protection of the central nervous system against the adverse effects of toxic environmental factors, not endogenous, but protecting against the formation of aggregates and protein deposits in cells in healthy individuals to such an extent that there is no slow loss of cognitive function and memory in healthy individuals leading to brain ageing.

Admittedly, a recent report [Hosseini SA, Salehi O, Keikhosravi F, Hassanpour G, Ardakani HD, Farkhaie F, Shadmehri S, Azarbayjani MA (2021) Mental health benefits of exercise and genistein in elderly rats. Exp Aging Res 22: 1-16] only suggests the possibility of improved memory and anxiety parameters following the use of genistein in combination with increased physical activity in ageing rats, nevertheless, these effects were mainly observed only following the joint application of the two methods (increased physical activity and intraperitoneal administration of genistein) and it is not known which factor has a significant effect - whether both at the same time, separately, synergistically, etc. Furthermore, genistein's effects are antioxidant and oestrogenic, indicating nullification of the toxic effects of various toxic substances, rather than protection against the formation of aggregates and deposits of proteinaceous substances in brain cells in the process of brain ageing - which is what the present invention is concerned with. Furthermore, the intraperitoneal administration of genistein to animals is described here.

In WO01/00215, compositions are described for improving memory and concentration in mammals with disorders associated with memory impairment. The composition comprises a combination of at least one phytoestrogen and at least one acetylcholinesterase inhibitor or any derivative, analogue, metabolite thereof. The composition may further comprise at least one estrogen. The method according to the invention comprises the simultaneous administration of at least one phytoestrogen and at least one acetylcholinesterase inhibitor in a therapeutically effective amount. Genistein is mentioned as an example among the phytoestrogen. The use of the composition in the prevention of the development of neurodegenerative diseases and cognitive dysfunction has also been described. However, the use of genistein was to protect against external substances adversely affecting the body once they enter the body through oxidative stress, possibly acting through the stimulation of oestrogen receptors. As a substance with antioxidant activity, genistein could protect cells against reactive oxygen species from the environment. However, the action of genistein itself as a prevention against the endogenous formation of deposits of misfolded proteins such as beta-amyloid in healthy people during physiological brain ageing has not been described. The antioxidant effects of genistein at low doses have been described.

US2005/0031651 describes the use of two ingredients, where the first agent targets the treatment of a disease associated with beta amyloid deposition. An additional use of antioxidants is described where genistein is mentioned among others. In this case, the invention described does not concern the prevention of memory and learning loss progressing with age, but the treatment of a pathophysiological condition. Furthermore, genistein is used as an antioxidant, so it would be intended to protect against the adverse effects of external (environmental) factors and not endogenous factors.

In WO2020/012175, a composition containing an effective amount of a combination of two or more ingredients is described, the listed ingredients being selected from a list where genistein is mentioned. The composition is for use in increasing cellular resistance to DNA damage, protection against oxidative stress, mitochondrial dysfunction or in improving DNA repair capacity. Also in this document, genistein is described as a compound with antioxidant properties, i.e. to protect against adverse effects of external (environmental) factors.

EP1774972 describes selenium-containing compositions (e.g. Sel-Plex) for use as a therapeutic or preventive treatment for neurodegenerative disease. The use of an antioxidant in a composition such as genistein is also described. In this case, the effect of selenium is quite different from that of genistein. On the other hand, genistein is listed as an antioxidant, i.e. a compound intended to protect against the adverse effects of external (environmental) factors, rather than endogenous factors such as the formation of intracellular protein deposits in healthy individuals.

Furthermore, a publication [Kaikhosravi F, Daryanoosh F, Koushkie Jahromi M, Nemati J. The Effect of High Intensity Interval Training with Genistein on Biomechanical Properties of Femur Bone in Elderly Female Rats. Jorjani Biomed J. 2020; 8: 51-59] described the effect of genistein in bone repair, increasing sex hormones in the elderly, and protecting against osteoporosis. However, this work points to the somatic - on somatic cells - effects of genistein, without pointing to central nervous system functions.

The direct effects of genistein shown earlier in the state of the art apply to the ageing of somatic tissues, the prevention of adverse effects of certain environmental factors and therapeutic effects in neurological diseases, but not to the ageing of the brain of healthy individuals.

It seems that the direct cause of age-related memory and learning impairment - understood as brain ageing in healthy individuals (and not with the presence of a specific neurodegenerative disease entity) is the formation of deposits of insoluble proteins, mainly β-amyloid, in neurons, leading to a gradual loss of nerve cell function [Sojkova J, Zhou Y, An Y, et al. Longitudinal patterns of β-amyloid deposition in nondemented older adults. Arch Neurol. 2011;68(5):644-649; Brown EE, Graff-Guerrero A, Houle S, et al. Amyloid deposition in semantic dementia: a positron emission tomography study. Int J Geriatr Psychiatry. 2016;31(9):1064-1074.].

The aim of the invention was therefore to develop a means of preventing the loss of memory and cognitive abilities in people as a result of the physiological processes of brain ageing in healthy people in order to prevent and reduce the accumulation of harmful insoluble aggregates and protein deposits in brain cells - neurons.

The objective is to prevent and reduce the formation of deposits of insoluble proteins and/or aggregates of insoluble proteins in the brain formed as a result of the physiological process of brain ageing as healthy people age, and thus to prevent loss of memory and cognitive functions in a group of people, i.e. healthy people where it is precisely the loss of memory and cognitive function that occurs in brain ageing as a result of the deposition of proteins and aggregates of other insoluble compounds in the brain as a result of the physiological ageing process, especially beta amyloid, but not people with neurodegenerative diseases. This is because, unlike the process of beta-amyloid deposition in the central nervous system in neurodegenerative diseases - Alzheimer's disease - in healthy people, the process differs in that there is no significant neuronal loss associated with neurodegeneration processes, as illustrated by the atrophy of increasingly large parts of the brain. In Alzheimer's disease, there is no physiological ageing of the brain as in healthy people with age, but there is a process of necrosis of neurons, i.e. atrophy of brain tissue . Unlike other uses of genistein, which is used in low doses as an antioxidant agent to protect cells from oxidative stress caused by external factors, the invention concerns the use of genistein to protect against endogenous (internal) processes of protein deposits leading to memory and cognitive loss in healthy people.

The invention, unlike the known ones, concerns the prevention of loss of memory and cognitive function in healthy individuals, and the preventive action consists in preventing the formation of deposits of insoluble proteins and their removal if previously formed, so that the function of the neural cells in the given range in healthy individuals is protected and thus the action of genistein is towards brain ageing.

The invention relates to the oral administration of genistein in healthy individuals in the medical use of preventing memory and cognitive loss of healthy individuals in brain ageing as a result of ageing processes in healthy individuals.

It should be pointed out that oral administration of genistein has a huge practical advantage over intraperitoneal administration when the compound is used in humans - the latter being unacceptable for routine use for prophylactic purposes.

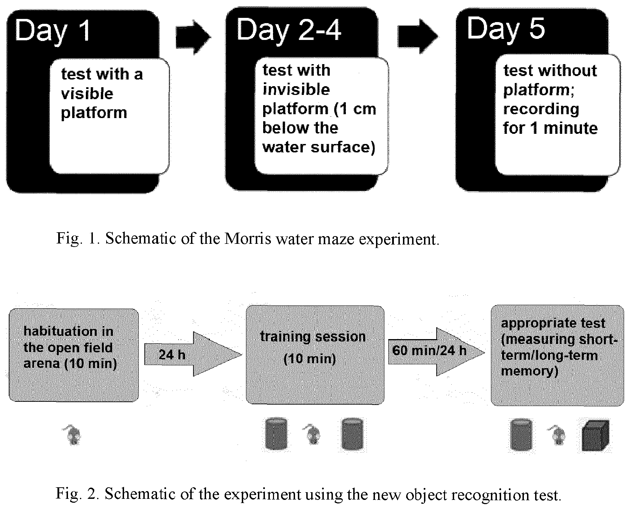

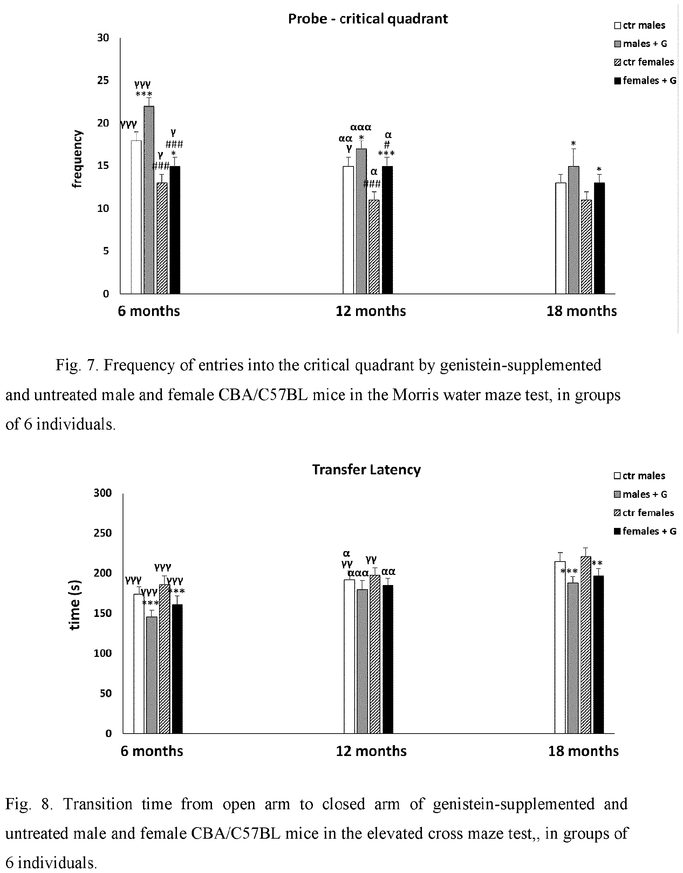

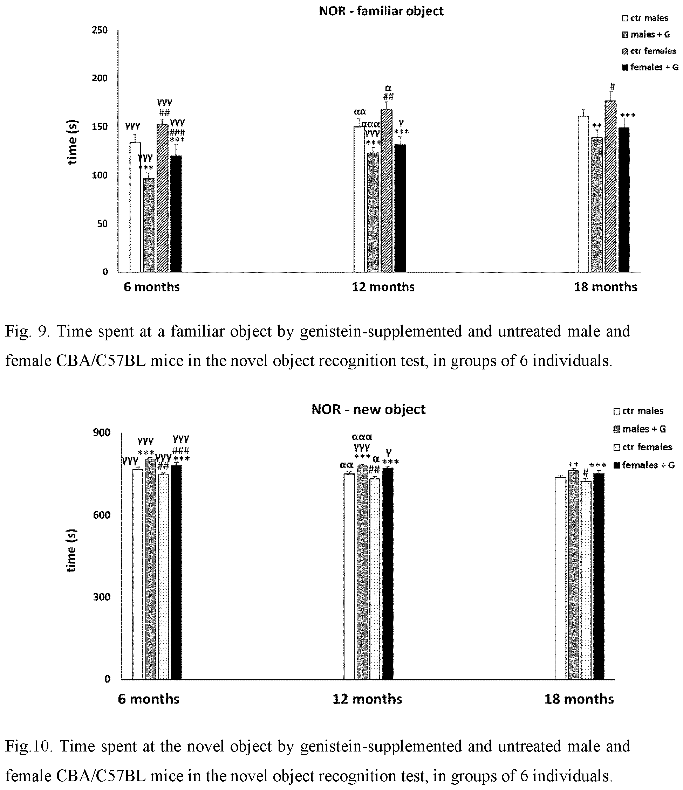

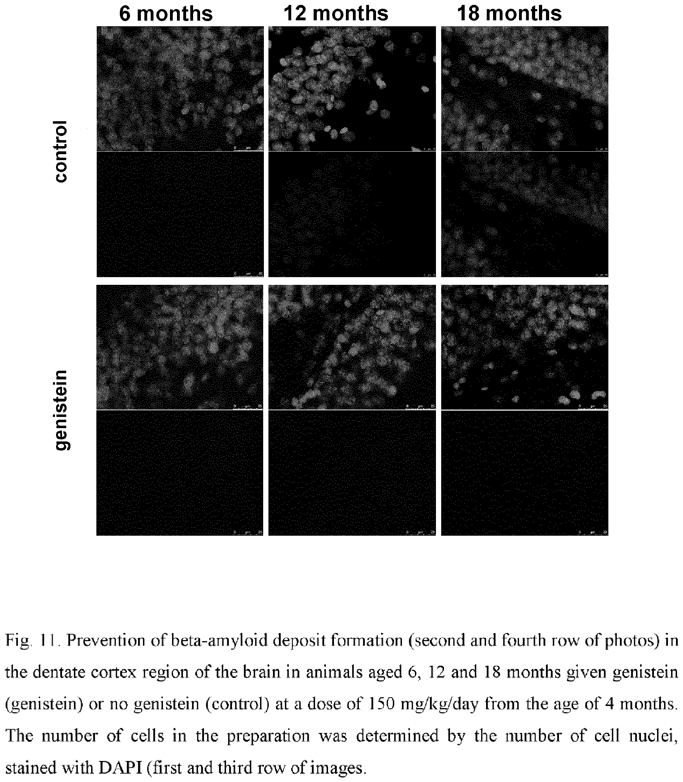

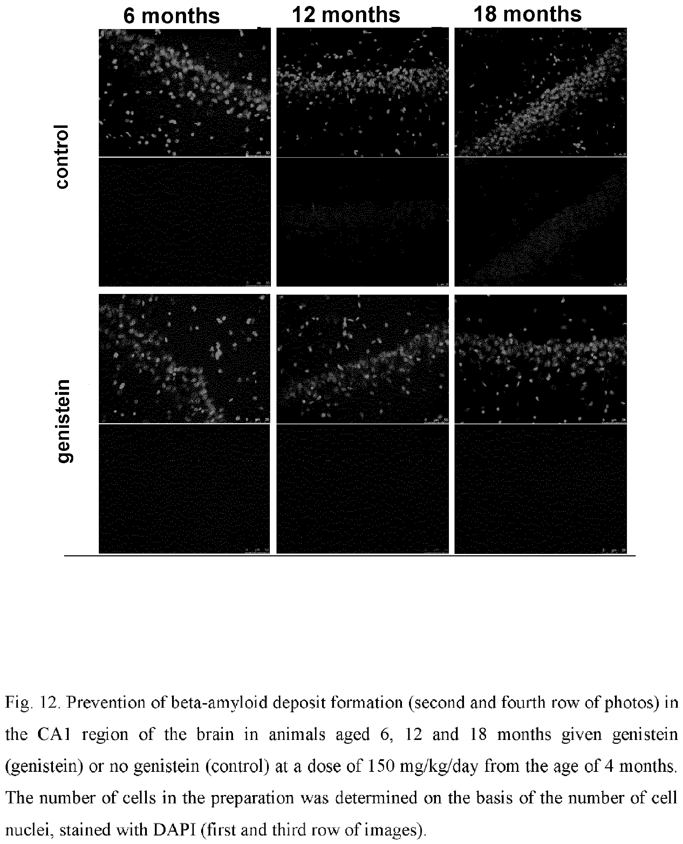

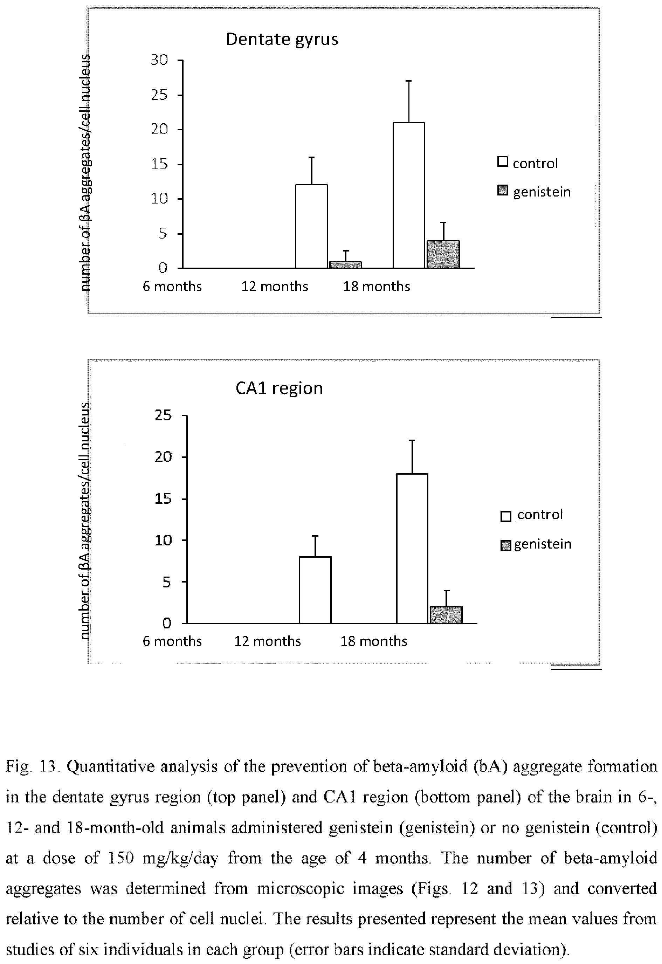

The invention is described in more detail in the examples, where Figs. 1 to 7 show the results of experiments using the Morris Water Labyrinth. These experiments allow the efficiency of the memory and cognitive (learning) abilities of the animals to be evaluated. In this example, the effect of the compound is shown, which confirms the protective effect of genistein on the animals' memory and learning abilities, which are reduced in older, healthy animals, but the administration of genistein prevents these adverse effects in such a way that the results obtained for older animals are comparable to those observed in young individuals. The absence of neurodegenerative diseases including Alzheimer's was confirmed and demonstrated by using lines of healthy animals, not genetically burdened or subjected to any procedures to induce the disease, and not showing neurodegenerative features in the form of atrophy of parts of the brain. Fig. 8 shows the results of an experiment with an elevated cross maze to estimate the animal's memory and academia in response to stress conditions. The use of genistein prevents the deterioration of performance in older animals, which is observed in individuals not given genistein. Figs. 9 and 10 show the results of novelty tests, which measure behavioural parameters of animals under conditions of being in a new environment. Older individuals show less learning ability, reflected by less interest in previously unfamiliar objects. Administration of genistein causes older animals to behave in a similar way to young animals. All three tests used to analyse the course of cognitive functions confirmed the prophylactic potential of genistein to prevent the occurrence of disorders of memory processes, the likelihood of which increases with age. The results presented in Figs. 11-13 show that genistein prevents the accumulation of β-amyloid deposits in the brains of older healthy animals (analogous β-amyloid accumulation in older healthy humans has been previously reported [Sojkova J, Zhou Y, An Y, et al. Longitudinal patterns of β-amyloid deposition in nondemented older adults. Arch Neurol. 2011;68(5):644-649; Brown EE, Graff-Guerrero A, Houle S, et al. Amyloid deposition in semantic dementia: a positron emission tomography study. Int J Geriatr Psychiatry. 2016;31(9):1064-1074.]). Beta-amyloid levels in the brains of older animals given genistein were significantly reduced compared to analogous animals not given genistein, approaching the results seen in young animals.

### Example

### Memory and cognitive parameters - cognitive

The analysis of the prophylactic potential of genistein was performed on male and female mice of strain CBA/C57BL (healthy mice) at the ages of, respectively: 6 months, 12 months and 18 months. The absence of neurodegenerative diseases including Alzheimer's was demonstrated by using lines of healthy animals, not genetically burdened or subjected to any procedures to induce the disease, and not showing neurodegenerative features in the form of atrophy of parts of the brain. According to the literature [Dutta S, Sengupta P. Men and mice: Relating their ages. Life Sci. 2016;152:244-248], 1 year in the life of a human corresponds to 9 days in the life of a mouse, hence the ages chosen for analysis are, per man: 20 years, 40 years and 60 years. Genistein supplementation was started when the animals were 4 months old. Isoflavone (genistein) was administered daily at a dose of 150 mg/kg/day (a dose considered safe)), orally in a volume of 0.2 ml via a 25 mm long metal gavage. The use of the gavage was to ensure that the desired dose was delivered orally to the mice. Cognitive parameters were assessed in the Morris water maze test and the results were confirmed in the elevated cross maze and the novel object recognition test.

The Morris water maze (MWM) is one of the most widely used tests to assess spatial memory in rodents. It is used in studies of neurodegenerative diseases and ageing. The learning impairments observed in the MWM have been shown to be independent of locomotor abnormalities, as locomotor limitations do not affect the swimming speed of the animals. The experimental set-up consisted of a black circular pool of water with a diameter of 150 cm and a depth of 60 cm. The experiment was recorded using a black and white analogue video camera connected to a processing device (EthoVision XT software, Noldus, Netherlands), which optically divided the pool into four equal parts and recorded the time the animal spent in each quadrant. The pool was equipped with a contrasting (landmark) point on the wall (above the water surface) and a circular platform of transparent plastic with a diameter of 10 cm placed centrally in the area of one of the quadrants (CQ). The experiment was conducted for 5 consecutive days at the same time (from 8.00 - 12.00), according to the following stages - shown in Fig. 1 - diagram of the experiment:
- Day 1 - training session - the mouse was placed in the water, with its snout facing the pool wall, in the quadrant of the pool opposite the platform. During the training session, the transparent platform was placed 1 cm above the water surface to allow the animals to consciously remember its location. If the mouse did not find the safe point (platform) within the set time of 60 s, it was gently manually directed to the platform, where it remained for 10 s before being transferred to the cage. If the mouse found the platform in less than 60 s, it remained on the platform for an additional 5 s until recording was completed.
- Day 2 - 4 - spatial memory test - platform placed in the pool about 1 cm below the water surface, (centrally in the same quadrant (CQ) as for the training session) in such a way that it became directly invisible to the animal. The experiment was started by placing the mouse in the water in the same way as the training session. During each session, the mouse swam in the water until it found a safe place (latency) - entering the flat surface of the platform - or until a time of 120 s elapsed. If the mouse did not find the platform within the set time, it was manually guided to locate it and remained on the platform for a further 10 s. During the course of the experiment, the following parameters were recorded to assess the progress of spatial memory processes, which were assumed to be as follows on successive days of the experiment (day 2-4): a reduction in the distance covered, an increase in the amount of time spent in the CQ quadrant, a reduction in the time required to find the platform (latency).
- Day 5 - memory test - platform removed from the pool (probe test). The experiment was started by placing the mouse in the same quadrant as on the previous days, facing the pool wall. Recordings were made for 1 min. In order to assess the correctness of the reference memory processes, the time to reach the critical quadrant in which the platform was located in the previous repetitions was tested. Each individual was subjected to one experimental session on a given day, consisting of four repetitions of recording, separated by a 10 min break in time.

The Elevated Cross Maze (EPM) test, is an anxiety response assessment method designed for rodents. It can also be used to analyse memory processes under anxiety conditions by recording the latency of the transition from the open to the closed arm. The experimental set-up consisted of a cross-shaped platform placed 50 cm above the ground. The EPM test was made of Plexiglas and consisted of two white open arms (50 cm × 10 cm) - aversive - evoking a sense of danger in the animals, and two black opposite arms surrounded by 40 cm high walls (closed). Joining the arms was a central white square (10 cm × 10 cm). Observed animal behaviour was recorded using a camera connected to an automatic tracking system (EthoVision XT, Noldus, Netherlands). After each measurement was performed on a given individual, the platform was cleaned with 70% ethanol and allowed to dry for 5 minutes to eliminate the influence of odour traces on the animal's responses. In each group, the test was performed at the same time of day (between 8.00 and 12.00). The test started by placing the mouse at one end of the open arm, with its back to the maze. During the recording, the transition latency, i.e. the transition time from the open (aversive) arm to the closed (safe) arm, was analysed to monitor the animal's memory processes.

The novel object recognition test (NOR), on the other hand, can be used to measure working memory, attention and anxiety and also to investigate the response to novelty in rodents. The experimental set-up consisted of a square arena (100 cm × 100 cm) with a white floor, divided by black lines into 25 equal squares, surrounded by uniform white walls 60 cm high, and a set of three objects - two identical and one different - two identical cups and one large wooden cube (difference in colour, shape and texture). The test was conducted from 8⁰⁰ - 12⁰⁰. Each measurement started with the mouse being placed in the corner of the arena (starting quadrant). Objects were placed at the opposite end of the arena, always in the same quadrants. After each recording, the surface of the arena was cleaned with 70% ethanol and allowed to dry (5 min) to eliminate the effects of odour traces on the behaviour of the mice. Animal activity was recorded using a video camera connected to an automatic tracking system (EthoVision XT, Noldus, Netherlands).

The test was performed in three series according to the scheme - Fig. 2 - diagram of the experiment:
• day 1 - 24 h prior to the test the animals are habituated (10 min) in an open field arena (no additional objects) to familiarise them with the experimental environment;
• Day 2 - training session - the animals are placed in an arena with two identical objects - exploration of the objects by the animals lasts 10 min. After 60 min to test short-term memory, a proper test is carried out, during which one of the objects is replaced by a new object of a different colour, texture;
day 3 - 24 h after the training session, a test is performed (to investigate long-term memory processes) by placing the animals in an arena with a familiar and a new object (exploration for 10 min). New object recognition test - a diagram of the experiment is shown in Fig. 2.

Due to the innate curiosity of mice, the NOR test does not require lengthy schedules of training sessions or the use of additional reinforcement. Animals should naturally, after recognising a familiar object, spend more time with an object they recognise as new.

A preference index was used to analyse the results, which is expressed as the amount of time spent recognising a new object (B), relative to the amount of time spent recognising a known object (A). With undisturbed cognitive processes, the relationship is maintained: time spent recognising a known object (A) < time spent recognising a new object (B).

In order to avoid the influence of one test on the results of the next, all mentioned tests were separated by a 1-day break, during which the animals were not subjected to any analysis.

### Morris water maze - latency to platform

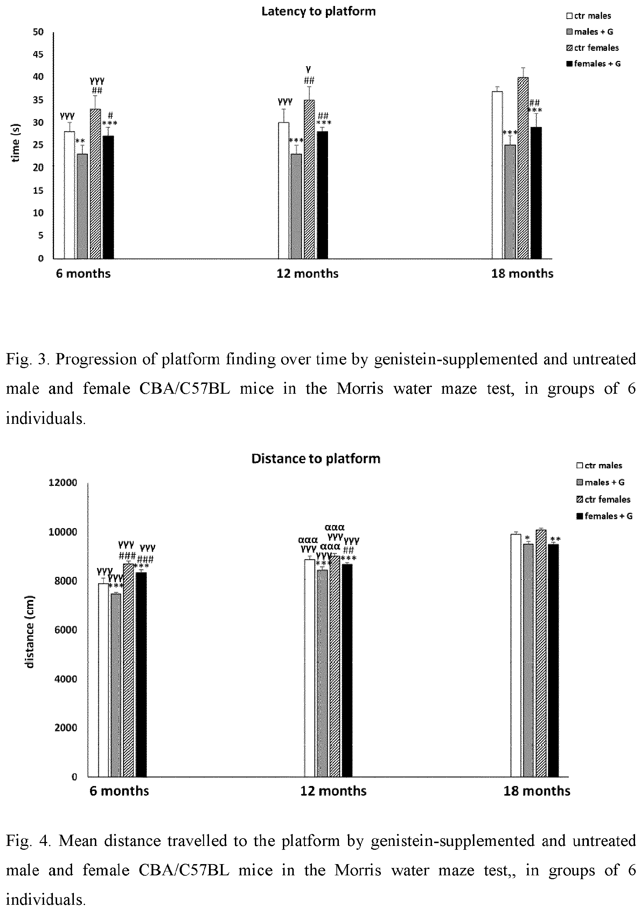

Explanation from fig. 3:
*** - p<0.001; ** - p<0.01 - vs. ctr
## - p<0.01; # - p<0.05 - vs. males
γγγ - p<0.001; γ - p<0.05 - vs. 18 mo.

Fig. 3 shows that in older animals (18 months old) given genistein daily from the age of 4 months, platform finding time parameters, one measure of memory performance, were at the level of young animals (6 months old), while in older control animals they deviated significantly to the disadvantage of young animals.

### Morris water maze - distance to platform

Explanation from fig.4:
*** - p<0.001; ** - p<0.01; * - p<0.05 - vs. ctr
### - p<0.001; ## - p<0.01 - vs. males
γγγ - p<0.001 - vs. 18 m-c
ααα - p<0.001 - vs. 6 m-c

Fig. 4 shows that in older animals (18 months old) given genistein daily from the age of 4 months, the parameters of the distance needed to find the platform, which is one measure of learning efficiency, were significantly better than in control animals.

### Morris water maze - time in the critical quadrant (CQ)

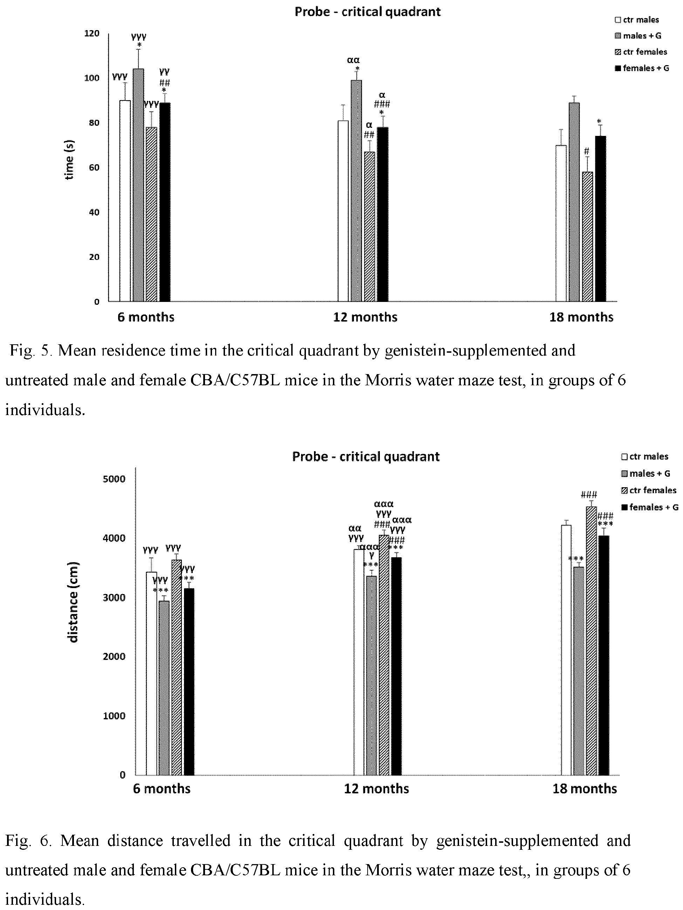

Explanation from fig.5:
* - p<0.05 - vs. ctr
### - p<0.001; ## - p<0.01; # - p<0.05 - vs. males
γγγ - p<0.001; γγγ - p<0.01 - vs. 18 mo.
αα - p<0.01; α - p<0.05 - vs. 6 mo.

Fig. 5 shows that in animals given genistein daily from the age of 4 months, the parameters of time spent in the critical quadrant, one measure of learning performance, were significantly better than in control animals.

### Morris water maze - distance in critical quadrant (CQ)

Explanation from fig.6:
*** - p<0.001 - vs. ctr
### - p<0.001 - vs. males
γγγ - p<0.001; γ - p<0.05 - vs. 18 mo.
ααα - p<0.001; ααα - p<0.01 - vs. 6 mo.

Fig. 6 shows that in animals given genistein daily from the age of 4 months, the parameters of mean distance travelled in the critical quadrant, one measure of memory performance, were significantly better than in control animals.

### Morris water maze - critical quadrant (CQ) inflow rate

Explanation from fig.7:
*** - p<0.001; * - p<0.05 - vs. ctr
### - p<0.001; # - p<0.05 - vs. males
γγγ - p<0.001; γ - p<0.05 - vs. 18 mo.
ααα - p<0.001; α - p<0.05 - vs. 6 mo.

Fig. 7 shows that in animals given genistein daily from the age of 4 months, the frequency of entries into the critical quadrant, one measure of memory performance, was significantly higher than in control animals.

### Summary of results from Morris water maze

In all parameters of the MWM, the statistically significant prophylactic potential of genistein in preventing the gradual (physiological) loss of cognitive function, which increases with age, is apparent. The positive effect on memory processes applies to both males and females, in whom studies have shown that dementia disorders appear more rapidly and are more difficult to treat. The beneficial effect of isoflavone is manifested in the test by a reduction in the time to find the platform, a reduction in the distance covered to reach the platform, an increase in the time spent in the critical quadrant, and an increase in the frequency of entering the critical quadrant by genistein-treated mice compared to unsupplemented animals. Improvements in the functioning of memory processes are also evident in older animals (18 months), whose parameters do not differ or are similar to those recorded in young animals (6 months).

### Elevated cross maze (EPM) - transition latency

Explanation from fig.8:
*** - p<0.001; ** - p<0.01 - vs. ctr
γγγ - p<0.001; γγγ - p<0.01 - vs. 18 mo.
ααα - p<0.001; αα - p<0.01; α - p<0.05 - vs. 6mc

Fig. 8 shows that in older animals (18 months old) given genistein daily from the age of 4 months, the parameters of the transition time from the open arm to the closed arm, which is one measure of memory performance under stress, were at levels similar to those observed in young animals (6 months old), whereas in older control animals they deviated significantly to the disadvantage of young animals.

In the EPM test, genistein significantly affected the transition time from the open (aversive) arm to the closed arm in mice that received it. Regardless of the age of the animals that were analysed, the transition latency was reduced after isoflavone treatment. Similarly, as with the parameters from the MWM, older mice were not significantly different from younger animals. Prophylactic potential is evident for animals of both sexes.

### New object recognition test - time with a known object

Explanation from fig.9:
*** - p<0.001; ** - p<0.01 - vs. ctr
### - p<0.001; ## - p<0.01; # - p<0.05 - vs. males
γγγ - p<0.001; γ - p<0.05 - vs. 18 mo.
ααα - p<0.001; αα - p<0.01; α - p<0.05 - vs. 6 mo.

Fig. 9 shows that in older animals (18 months old) given genistein daily from the age of 4 months, the parameters of time spent with a familiar object, which is one measure of cognitive performance, were at the level of young animals (6 months old), whereas in older control animals they deviated significantly to the detriment of young animals (measured time is inversely proportional to cognitive ability).

### New object recognition test - time at new object

Explanation from fig. 10:
*** - p<0.001; ** - p<0.01 - vs. ctr
### - p<0.001; ## - p<0.01; # - p<0.05 - vs. males
γγγ - p<0.001; γ - p<0.05 - vs. 18 mo.
ααα - p<0.001; αα - p<0.01; α - p<0.05 - vs. 6 mo.

Fig. 10 shows that in older animals (18 months old) given genistein daily from the age of 4 months, the parameters of time spent with a novel object, which is one measure of cognitive performance, were at the level of young animals (6 months old), whereas in older control animals they differed significantly to the detriment of young animals (measured time is directly proportional to cognitive ability).

### Summary of results from the new object recognition test

Analogous observations apply to both parameters in the NOR test. Animals that were not given genistein spent more time with a familiar object and less time with a new object. The beneficial effects of genistein are evident in female and male mice of all ages analysed.

All three tests used to analyse the course of cognitive function confirmed the preventive potential of genistein to prevent the onset of memory process disorders, the likelihood of which increases with age.

### Quantitative assessment of beta-amyloid deposits in brain structures

To determine the effect of genistein on the formation of beta-amyloid deposits in ageing animals, the level of beta-amyloid in selected brain structures (- dentate acetabulum and CA1 region) was determined. The experiments were performed analogously to the behavioural studies. Brain structures were analysed in animals at multiple 6, 12 and 18 months. After euthanasia, microscope slides stained with DAPI (control staining of cell nuclei) were prepared and beta-amyloid deposits were visualised using specific antibodies directed against this protein, and secondary antibodies conjugated to a fluorescent dye. The slides were analysed using a fluorescence microscope, observing and determining the number of beta-amyloid aggregates, and the values obtained were then normalised per number of cell nuclei (indicating the number of cells in a given slide).

As shown in Fig. 11-13, the appearance and accumulation of beta-amyloid deposits is observed with age in healthy laboratory animals. The use of genistein, in the form of daily oral administration at a dose of 150 mg/kg/day, from the age of 4 months until the end of the experiments (as described above when presenting the behavioural studies) resulted in a very significant reduction in the formation of beta-amyloid deposits in the brains of the animals. The level of these deposits in the 18-month-old animals given genistein was significantly lower than in the control 12-month-old animals and only slightly higher than in the control 6-month-old animals. These results indicate that the use of genistein effectively prevents the accumulation of beta-amyloid deposits in the brain, which has a protective effect on this organ and may prevent the neurological effects of ageing - such as a decrease in memory and cognitive parameters. In healthy animals (as in humans), only the accumulation of beta-amyloid can be clearly seen with age. In patients with various diseases, other deposits (tau protein, glycosaminoglycans, etc.) can be easily detected, but in healthy animals, with ageing, only beta-amyloid is detectable in such quantities that comparisons can then be made with animals given various preparations. Thus, beta-amyloid was considered as a model protein to be assessed. Other proteins are also deposited in old mice, but they are so diverse that specific antibodies cannot be used to detect them. If, on the other hand, one wanted to detect all of them at once, one would also detect non-pathogenic proteins that are needed for cell function, so the results would be inconclusive. Beta-amyloid is therefore an excellent 'model' in these studies, and at the same time it is one of the main proteins toxic to nerve cells, responsible for loss of memory and cognitive function.

Figs. 11, 12 and 13 show that in older animals (18 months old) given genistein daily from the age of 4 months, the level of beta-amyloid deposits (the main insoluble protein that accumulates with age in the brain and is one of the primary causes of progressive memory and cognitive loss) was significantly reduced, approaching the results observed in young animals (6 months old), while in older control animals it differed significantly to the disadvantage of young animals.

## Claims

1. Genistein for use in the prevention of memory and cognitive loss in healthy people as a result of the gradual brain ageing processes that occur physiologically in healthy people with age, while the genistein acting as an agent to prevent and reduce the deposition of harmful insoluble aggregates and protein deposits in the brain, especially beta-amyloid, and is administered orally.
